# EUROPÄISCHE PATENTSCHRIFT

(11) **EP 4 021 298 B1**
(45) Veröffentlichungstag und Bekanntmachung des Hinweises auf die Patenterteilung: **01.10.2025**
(21) Anmeldenummer: 20735066.1
(22) Anmeldetag: 10.06.2020
(51) Int. Cl.: A61B 5/117, B60W 60/00, G01C 21/34, A61B 5/11

(54) **FORTBEWEGUNGSMITTEL, VORRICHTUNG UND VERFAHREN ZUM POSITIONIEREN EINES AUTOMATISIERT FAHRFÄHIGEN FORTBEWEGUNGSMITTELS**
MEANS OF TRANSPORT, DEVICE AND METHOD FOR POSITIONING AN AUTOMATICALLY OPERABLE MEANS OF TRANSPORT
MOYEN DE TRANSPORT, DISPOSITIF ET PROCÉDÉ DE POSITIONNEMENT D'UN MOYEN DE TRANSPORT ACTIONNABLE AUTOMATIQUEMENT

(30) Priorität: 29.08.2019 DE 102019212998
(43) Veröffentlichungstag der Anmeldung: 06.07.2022
(73) Patentinhaber: VOLKSWAGEN AKTIENGESELLSCHAFT, 38440 Wolfsburg (DE)
(72) Erfinder: EHMANN, Sebastian, 38302 Wolfenbüttel (DE); RICHTER, Robin, 38518 Gifhorn (DE)
(86) Internationale Anmeldenummer: PCT/EP2020/066007
(87) Internationale Veröffentlichungsnummer: WO 2021/037409

(56) Entgegenhaltungen:
- EP-A1- 3 579 158
- WO-A1-2018/142528
- WO-A1-2019/206478
- US-A1- 2017 153 714
- US-A1- 2018 039 917
- US-A1- 2018 338 229

## Beschreibung

Die vorliegende Erfindung betrifft eine Vorrichtung, ein Fortbewegungsmittel sowie ein Verfahren zum Positionieren eines automatisiert fahrfähigen Fortbewegungsmittels.

Im Stand der Technik werden erhebliche Anstrengungen unternommen, Fahrzeuge mit einem hohen Maß an Fahrautomation zu produzieren. Diese werden unter anderem von Privatkunden und in Fahrzeugflotten von Unternehmen genutzt; auch eine Nutzung für Transportdienstleistungen, wie autonome Taxidienste oder geteilt genutzte Fahrzeuge (Car Sharing), wird angestrebt. Fortbewegungsmittel, die für Transportdienstleistungen genutzt werden, zeichnen sich insbesondere dadurch aus, dass sie einem Fahrgast nur kurzfristig zuordenbar sind. Die Durchsetzung und Nutzung automatisiert fahrfähiger Fahrzeuge, insbesondere in den letztgenannten Bereichen, ist von der Nutzerakzeptanz, die unter anderem mit dem Komfort der Nutzung einhergeht, automatischer Fortbewegungsmittel abhängig. Ein hoher Komfort der Nutzung eines automatisiert fahrfähigen Fahrzeugs kann beispielsweise durch Systeme und Verfahren erreicht werden, die die Ausführungsarten und Funktionalitäten des Fortbewegungsmittels spezifisch auf die Wünsche und Bedürfnisse eines Insassen anpassen. Hierunter kann beispielsweise eine geeignete Sitzplatzzuordnung, eine vordefinierte Fahragilität des Fortbewegungsmittels, eine Einstellung eines Sitzes oder einer Lüftung verstanden werden. Die Einstellung erfolgt auf Basis von definierbaren oder erlernten Einstellungen, die einem Insassen zugeordnet sind, und ist insbesondere für ein einem Insassen fest und längerfristig zuordenbares Fortbewegungsmittel möglich.

DE 10 2007 023 140 A1 offenbart ein System, welches in ein autonomes Fahrzeug integriert wird und zur Authentifizierung von Fahrgästen dient. Weiterhin ist hier ein Kommunikationsmodul offenbart, welches eine personalisierte Kommunikation zwischen dem Fahrzeug und Fahrgästen ermöglicht.

WO 2017 / 005 495 A1 offenbart ein System zur Sitzplatzkennzeichnung in einer Transporteinheit, wobei die Sitzplatzzuordnung für jeden Fahrgast zur Orientierung sichtbar angezeigt wird und sich u.a. an Reservierungsdaten und dem Streckenverlauf orientiert.

DE 10 2017 119 577 A1 offenbart allgemein eine zeitliche Planung autonomer Fahrzeuge mittels mobiler Geräte.

DE 10 2018 212 901 A1 offenbart ein Verfahren zur Ermittlung einer Halteposition für ein automatisiert betriebenes Fahrzeug, durch welches das automatisiert betriebene Fahrzeug in einem vorderen Bereich einer Haltebucht einer Bushaltestelle zum Stehen gebracht werden kann.

DE 10 2018 210 450 A1 offenbart ein Verfahren zum Ausgeben von Steuersignalen zur Steuerung eines automatisierten Fahrzeugs, bei welchem der Sitzplatz eines Fahrzeuginsassen innerhalb des Fahrzeugs darüber entscheidet, wie sich das Fahrzeug am Zielort basierend auf empfangenen Umfelddaten positioniert.

DE 10 2018 113 781 A1 offenbart ein Verfahren zum Bereitstellen einer verbesserten Fahrgastnutzung eines autonomen Fahrzeugs, bei welchem gegebenenfalls in einem Zielbereich befindliche Ausstiegshindernisse vom Fahrzeug erkannt und dem Insassen (Fahrgast) gemeldet werden.

DE 10 2018 119 239 A1 betrifft ein Benutzererkennungssystem und -verfahren für autonome Fahrzeuge, mittels dessen unabhängig vom Vorhandensein eines elektronischen Drahtloskommunikationsendgerätes Anwendergesten von Passanten durch die Sensorik eines autonomen Fortbewegungsmittels erkannt und als "Heranwinken"-Geste erkannt werden. Auf diese Weise können Passanten ohne die Verwendung tragbarer Drahtloskommunikationsendgeräte autonome Fortbewegungsmittel / Taxis heranwinken.

DE 10 2017 214 855 A1 betrifft ein Verfahren zur Bestimmung eines Haltepunktes für ein hoch- und/oder vollautomatisiert betriebenes Fahrzeug. Bei dem Verfahren werden Gefahrenwerte in wenigstens zwei Bereichen in der unmittelbaren Umgebung eines Zielortes ermittelt und ein Haltepunkt unter Berücksichtigung der ermittelten Gefahrenwerte bestimmt.

DE 10 2017 120 972 A1 betrifft ein Fahrgastabholsystem und -verfahren unter Verwendung eines autonomen Shuttle-Fahrzeugs, bei welchem ein Standort eines zukünftigen Fahrgasts auf Grundlage eines Signalaustausches zwischen dem Fahrzeug und der elektronischen Vorrichtung des zukünftigen Fahrgastes bestimmt wird.

DE 10 2018 132 140 A1 offenbart ein Verfahren zum Unterstützen des Beladens eines Fahrzeugs mit einem Rollstuhl, bei welchem ein Lidar-Sensor verwendet wird, um zu ermitteln, dass ein Benutzer innerhalb eines Schwellenabstandes von dem Fahrzeug positioniert ist und im Ansprechen darauf eine Umfeldleuchte eine Lichtprojektion nach unten neben das Fahrzeug erzeugt.

US 2018 / 039917 A1 offenbart ein Verfahren zum automatisierten Abholen eines zukünftigen Insassen an einer vordefinierten Position mittels eines Fortbewegungsmittels. Auf Basis einer reservierten Sitzposition innerhalb des Fortbewegungsmittels wird das Fortbewegungsmittel derart beim zukünftigen Insassen zum Stillstand gebracht, dass dieser einen größtmöglichen Komfort zum unmittelbaren Besetzen der Sitzposition erfährt.

Ausgehend vom vorgenannten Stand der Technik ist es eine Aufgabe der vorliegenden Erfindung, die Nutzungsakzeptanz automatisiert fahrfähiger Fortbewegungsmittel zu erhöhen, indem der Komfort insbesondere beim Ein- und Ausstieg des Fahrgastes erhöht wird.

Die vorstehend genannte Aufgabe wird erfindungsgemäß durch ein Verfahren zum Positionieren eines automatisiert fahrfähigen Fortbewegungsmittels nach Anspruch 1, eine ensprechende Vorrichtung nach Anspruch 11 und ein Fortbewegungsmittel zum Positionieren eines automatisiert fahrfähigen Fortbewegungsmittels nach Anpspruch 13 gelöst. Weitere Ausgestaltungen bzw. Weiterbildungen der Erfindung sind Gegenstand zusätzlicher Ansprüche 2-10 und 12. Die Positionierung erfolgt insbesondere anhand einer Sitzplatzzuordnung oder anhand erfasster Ausrüstungsgegenstände des Nutzers Das Fortbewegungsmittel kann beispielsweise ein PKW, Transporter, LKW, Luft- und/oder Wasserfahrzeug sein.

In einem ersten Schritt wird ein zukünftiger Insasse erkannt. Hierfür befindet sich das Fortbewegungsmittel in der Nähe (insbesondere < 10 m Abstand) des zukünftigen Insassen und es werden insbesondere sensorisch ermittelte Daten, die vorzugsweise durch im Fortbewegungsmittel zur Umgebungserkennung integrierte Sensoren aufgenommen werden, oder Daten eines tragbares Anwenderendgerätes des zukünftigen Insassen genutzt. Die Erkennung erfolgt vorzugsweise durch eine logische Zuordnungsprüfung mit in einer internen oder externen Datenbank gespeicherten Daten. Mit anderen Worten erfolgt eine Identifikation eines erwarteten Insassen. Im Ansprechen auf die Identifikation des zukünftigen Insassen werden vorzugsweise die vorbestimmten Sitzplatzinformationen mit den Insassendaten verknüpft. Ein Insasse eines Fortbewegungsmittels kann ein Fahrgast und/oder Führer eines Fortbewegungsmittels sein. In einem zweiten Schritt wird die aktuelle Position des identifizierten zukünftigen Insassen ermittelt. Zur Ermittlung der Position des zukünftigen Insassen befindet dieser sich vorzugsweise in unmittelbarer Umgebung des Fortbewegungsmittels und es besteht vorzugsweise eine Sichtverbindung zwischen dem Fahrzeug und dem Insassen. Hierfür werden insbesondere sensorisch ermittelte Daten, die durch im Fortbewegungsmittel zur Umgebungserkennung integrierte (insbesondere optische) Sensoren, auch als "Umgebungssensorik" bezeichnet, aufgenommen werden, oder Positionsdaten eines tragbaren Anwenderendgerätes des zukünftigen Insassen genutzt. Sensoren zur Umgebungserkennung können insbesondere Kameras zur Verkehrsschilderkennung und/oder zur Detektion von Fußgängern und/oder zur Einparkhilfe genutzte Ultraschall-Sensoren, vor allem Kameras, sein. In Abhängigkeit der aktuellen Position des zukünftigen Insassen wird eine Halteposition des Fortbewegungsmittels festgelegt. Die Halteposition zeichnet sich insbesondere durch eine relative Positionierung des automatisiert fahrfähigen Fortbewegungsmittels in Bezug auf die Position des zukünftigen Insassen aus und ist auch als optimale Positionierung zu bezeichnen. Die optimale Positionierung ist weiterhin dadurch gekennzeichnet, dass der zukünftige Insasse nach abgeschlossener Positionierung einen besseren Zugriff auf eine vordefinierte Tür des Fortbewegungsmittels hat als auf andere. Ein besserer Zugriff kann sich durch eine bessere Erreichbarkeit auszeichnen, worunter eine kurze Strecke zwischen der aktuellen Position des zukünftigen Insassen und der vordefinierten Tür verstanden wird. In die Festlegung der Positionierung können vordefinierte Referenzen eingehen, worunter beispielsweise fahrzeugspezifische Parameter, insbesondere Geometrien und Abmessungen, Positionen von Türgriffen etc. des Fortbewegungsmittels, oder Umgebungsparameter des Ein- oder Ausstiegspunktes verstanden werden. Auf Basis eines entsprechenden Signals erfolgt die Positionierung des Fortbewegungsmittels, somit wird der Anwenderkomfort erhöht.

Die Unteransprüche zeigen bevorzugte Weiterbildungen der Erfindung.

Die optimale Positionierung des Fortbewegungsmittels am Einstiegspunkt eines Insassen ergibt sich insbesondere aus einer optimalen Erreichbarkeit des vorgesehenen Zugriffspunktes des zukünftigen Insassen in Bezug auf eine vordefinierte, also eine ihm zugeordnete, Tür. Zur Zuordnung einer Tür, wobei als eine Tür alle fahrgastrelevanten Öffnungen des Fortbewegungsmittels, also alle Fahrgasttüren und Gepäckfachabdeckungen, wie beispielsweise eine Kofferraumklappe, verstanden werden, erfolgt z.B. eine automatische Auswertung, ob Ausrüstungsgegenstände auf Grund ihrer Abmessungen in einem Gepäckfach verstaut werden sollen oder müssen oder der zukünftige Insasse direkt einen ihm zugeordneten Sitzplatz einnehmen wird. Auf Grund der zugeordneten Tür wird in Verbindung mit der aktuellen Position des zukünftigen Insassen eine wegoptimierte Festlegung der Positionierung des Fortbewegungsmittels vorgenommen. So unterscheidet sich die Positionierung des Fortbewegungsmittels beispielsweise im Falle einer Zuordnung eines Sitzplatzes im vorderen Bereich des Fortbewegungsmittels, bei der beispielsweise ein Halten auf Höhe der B-Säule veranlasst wird, von einer Positionierung im Falle einer Zuordnung eines Sitzplatzes im hinteren Bereich des Fortbewegungsmittels, bei der beispielsweise ein Halten auf Höhe der C-Säule veranlasst wird, oder einer Positionierung, die auf Grund eines Ausrüstungsgegenstandes derart erfolgt, dass der zukünftige Insasse den Ausrüstungsgegenstand nach der Positionierung des Fortbewegungsmittels unmittelbar in ein Gepäckfach eingeben kann und bei der beispielsweise ein Halten auf Höhe des Fahrzeughecks veranlasst wird. In einem handelsüblichen, sogenannten 5-Türer gibt es entsprechend 4 Fahrgasttüren und eine Gepäckfachtür (auch "Heckdeckel" oder "Kofferraumklappe"). Nach der obenstehenden Systematik ist die Beifahrertür die dem Beifahrersitz nächstliegende Tür, die sich wiederum auf der rechten Fahrzeugseite zwischen der A- und B-Säule des Fahrzeugs befindet. Weitere fahrzeugspezifische Zuordnungspunkte und Logiken sind denkbar und haben keinen einschränkenden Charakter. Eine optimale Erreichbarkeit des vorgesehenen Zugriffspunktes des zukünftigen Insassen zeichnet sich insbesondere durch die kürzeste Wegstrecke zwischen der ihm zugeordneten Tür und der Position des wartenden Insassen aus, wobei idealerweise fahrzeugspezifische Geometrien und Abmessungen, wie beispielsweise das Öffnungsfeld der Tür, in die Berechnungen der optimalen Positionierung einbezogen werden. Eine optimale Positionierung des Fortbewegungsmittels am Ausstiegspunkt eines Insassen zeichnet sich insbesondere dadurch aus, dass der Insasse das Fortbewegungsmittel möglichst direkt von seinem Sitzplatz gefahrlos verlassen kann. In die Ermittlung der optimalen Positionierung können weiterführend Umgebungsparameter des Ein- und Ausstiegspunktes, wie zum Beispiel die Lage und Ausstattung des Ein- oder Ausstiegspunktes, der beispielsweise an einer Straße, an einem Bürgersteig oder auf einem Parkplatz liegen und verschiedene Elemente, wie beispielsweise eine Rampe enthalten kann, einfließen. Ist eine Positionierung zu bestimmen, die sich auf mehr als einen zukünftigen Insassen bezieht, die an einer Einstiegsposition zusteigen oder an einer Ausstiegsposition das Fortbewegungsmittel verlassen, so wird vorzugsweise die arithmetische Mittelposition der wartenden Insassen ermittelt und zur Ermittlung der kürzesten, mittleren Wegstrecke herangezogen bzw. ein Minimum der Summe der Entfernungen sämtlicher zusteigender Insassen vom Fahrzeug ermittelt und die zugehörige Position des Fortbewegungsmittels als Positionierungsdaten für das Fortbewegungsmittel verwendet. Somit können mehrere Haltevorgänge an einem "globalen" Einstiegsort bzw. Ausstiegsort vermieden bzw. zusammengefasst werden.

Eine Positionierung des Fortbewegungsmittels auf Grund eines zugeordneten Sitzplatzes erfolgt insbesondere unter Beachtung der dem zukünftigen Sitzplatz zugeordneten Tür, der Fahrzeuggeometrie sowie auf Basis von Umgebungsparametern am Ein- und Ausstiegspunkt. Ist keine direkte Erreichbarkeit der zugeordneten Tür vom Wartepunkt des Insassen möglich, wenn dieser beispielsweise am Straßenrand wartet und ihm, im Falle von Rechtsverkehr, ein Sitzplatz auf der linken Fahrzeugseite zugeordnet ist, so wird das Fortbewegungsmittel vorzugsweise unter Wahrung der kürzesten Wegstrecke zwischen wartendem Insassen und dem Einstiegspunkt positioniert, also zum Beispiel unter Beachtung der Fahrzeuggeometrie mit der vorderen rechten oder hinteren rechten Ecke des Fortbewegungsmittels neben dem wartenden Insassen. Die Zuordnung der Türen zu den Sitzplätzen kann vordefiniert sein und enthält dann eine eineindeutige Zuordnung eines Sitzplatzes zu einer Tür oder eine optionale Zuordnung eines Sitzplatzes zu verschiedenen Türen, wenn beispielsweise ein Mittelsitz sowohl von einer Tür auf der linken, als auch von einer Tür auf der rechten Fahrzeugseite gleich gut erreicht werden kann. Hierbei können automatisch bzw. sensorbasiert auch die aktuellen Belegungszustände weiterer Sitzplätze berücksichtigt werden, über welche der zukünftige Insasse den im zugeordneten Sitzplatz erreichen kann. Ein Sitzplatz zeichnet sich dadurch aus, dass er in einem Fortbewegungsmittel angeordnet ist und bestimmte Sitzplatzparameter aufweist. Sitzplatzparameter können unterschiedlichen Merkmalsklassen zugeordnet sein. Eine Merkmalsklasse kann allgemeine Parameter zum Sitzplatz enthalten und/oder Parameter, die eine Ausrüstung eines Sitzplatzes beschreiben und/oder Parameter, die eine Einrichtung eines Sitzplatzes beschreiben. Die Merkmalsklassen haben keinen einschränkenden Charakter und dienen lediglich der besseren Verständlichkeit der möglichen einflussgebenden Parameter zur Ermittlung eines zuzuordnenden Sitzplatzes. Zu den allgemeinen Sitzplatzparametern zählen mit anderen Worten insbesondere Informationen zur konkreten Position des Sitzplatzes innerhalb des Fortbewegungsmittels und seiner Ausrichtung beispielsweise in Bezug auf die Fahrtrichtung sowie eine Beschreibung seiner Zugänglichkeit über zumindest eine Tür. Die Informationen zur Zugänglichkeit können auch Informationen dazu enthalten, ob zur Erreichung des Sitzplatzes nach dem Zustieg ein anderer Sitzplatz überquert werden muss und/oder auf welcher Fahrzeugseite sich die dem Sitzplatz nächstliegende Tür befindet und/oder wo diese Tür in Bezug auf das Fortbewegungsmittel angeordnet ist. Über die allgemeinen Sitzplatzparameter hinausgehend können einem Sitzplatz weitere Merkmale zugeordnet werden, die zur insassen- und fahrauftragsspezifischen Sitzplatzzuordnung herangezogen werden können. Diese können sich auf eine sitzplatzspezifische Ausrüstung oder Einrichtung beziehen, wobei unter einer sitzplatzspezifischen Ausrüstung beispielsweise die Ausrüstung mit einem Kindersitz, einer Sitzheizung/-belüftung/-massagefunktion, einem Entertainmentsystem, einem Tisch, einem Becherhalter und/oder einer bestimmten Beinfreiheit, u.a. verstanden wird; und unter einer sitzplatzspezifischen Einrichtung beispielsweise eine definierte Einstellung der Rückenlehne, das Zurechtlegen gewünschter Lektüre, das Bereitstellen eines bestimmten Video- oder Audio-Entertainmentangebotes und/oder das Servieren von Speisen oder Getränken, u.a. verstanden wird. (Größe/Position/Ausstattung (Sitzheizung/Sitzbelüftung/Massagefunktion)/ Eignung für (Klein-) Kinder oder Babys) aufweist. Die Fahrzeuggeometrie gibt Aufschlüsse über die konkrete Geometrie des Fortbewegungsmittels sowie absolute Abmessungen, welche die Position der Türen des Fortbewegungsmittels, und insbesondere ihrer Bedienelemente, beschreiben und zur Berechnung des kürzesten Weges zwischen der aktuellen Position des zukünftigen Insassen und der zugeordneten Tür herangezogen werden können. Darüber hinaus können Umfeldparameter am Einstiegs- oder Ausstiegspunkt die Sitzplatzzuordnung beeinflussen. Wird beispielsweise ein Einstiegspunkt an einer mehrspurigen, stark befahrenen Straße gewählt, so muss der zugeordnete Sitzplatz in Ländern, in denen Rechtsverkehr gilt, idealerweise auf der rechten Fahrzeugseite angeordnet sein, um einen gefahrlosen Einstieg des Insassen zu gewährleisten; wohingegen bei einem Einstiegspunkt auf einem Parkplatz in den meisten Fällen alle Türen für den gefahrlosen Zustieg eines Insassen genutzt werden können. Darüber hinaus können infrastrukturelle Parameter, wie beispielsweise eine Rampe, Umfeldparameter am Einstiegs- und Ausstiegspunkt darstellen, die bei der Sitzplatzzuordnung beachtet werden.

Zur Verringerung der Komplexität der Ermittlung der optimalen Positionierung in Bezug auf einen einem zukünftigen Insassen zugeordneten Sitzplatz können die Sitzplätze in Sitzplatzreihen zusammengefasst werden, die jeweils denselben Zugangsparametern und Türen unterliegen. Insbesondere bei Kleinbussen sind eine Sitzreihe oder mehrere Sitzplatzreihen häufig über dieselbe Tür zugänglich. Insofern werden die Sitzplätze in diesem Fall einfach über eine ihnen (gemeinschaftlich) zugeordnete Tür charakterisiert und die dem zukünftigen Insassen zugeordnete Sitzposition automatische in Abhängigkeit einer Tür und ggf. ihres Öffnungsfeldes zugeordnet.

Ein Insasse kann ein oder mehrere Ausrüstungsgegenstände mit sich führen. Die datentechnisch verfügbare Fahrzeuggeometrie gibt dabei Aufschlüsse über die konkrete Geometrie des Heckbereiches, an dem zumeist die Gepäckfachtür angeordnet ist, sowie die Abmessungen des Fortbewegungsmittels, die zur Berechnung des kürzesten Weges zwischen der aktuellen Position des zukünftigen Insassen und der Gepäckfachtür herangezogen werden können. Liegen am Ein- oder Ausstiegspunkt beispielsweise bauliche Restriktionen vor, die die Umsetzung des geplanten Gepäckablageprozesses betreffen, fließen diese in die Positionierungsplanung ein. Als Ausrüstungsgegenstände sind insbesondere Gepäckstücke, beispielsweise Koffer oder Rucksäcke, Kinderwagen, Gehhilfen, Rollstühle oder Regenschirme zu verstehen. Ein im Gepäckfach mitzuführender Ausrüstungsgegenstand kann automatisch, beispielsweise sensorisch, insbesondere kamerabasiert, und insbesondere unter Verwendung einer digitalen Objektdatenbank oder per Eingabe ermittelt werden, indem der zukünftige Insasse entsprechende Eingabe- und/oder Auswahlfelder in einem Frontend eines digitalen Buchungssystems nutzt oder die Information durch einen Vermittlers zum Beispiel an einem Buchungsschalter eingegeben wird.

Zur Vereinfachung des Zusteigens, Aussteigens oder Ein- oder Ausladens eines Ausrüstungsgegenstandes kann nach der Positionierung eine automatische Entriegelung der vorbestimmten, und somit logisch mit dem Ein- oder Aussteigen bzw. Ein- oder Ausladen verknüpften, Tür erfolgen. Entsprechend kann der (zukünftige) Insasse die vorbestimmte Tür öffnen und ein- bzw. aussteigen oder seinen Ausrüstungsgegenstand in ein Gepäckfach einlegen oder aus diesem entnehmen. Die Tür kann auch selbsttätig, das heißt automatisiert, geöffnet und/oder aufgeschwenkt werden. Hierdurch wird eine weitere Komforterhöhung erreicht, da der (zukünftige) Insasse das Fortbewegungsmittel hands-free (freihändig), das heißt, ohne dass er händisch eingreifen muss, betreten bzw. verlassen bzw. seinen Ausrüstungsgegenstand hands-free (freihändig) in das Gepäckfach eines Fortbewegungsmittels einlegen oder aus dem Gepäckfach des Fortbewegungsmittels herausnehmen kann.

Die Zuordnung eines einem zukünftigen Insassen zuzuordnenden Sitzplatzes kann automatisiert durch ein technisches System oder manuell durch Zuweisung und Übergabe einer digitalen Information erfolgen. Die Zuordnungsentscheidung basiert dabei insbesondere auf einer sensorischen Erfassung des zukünftigen Insassen, einer Beachtung vordefinierter Referenzen und Umfeldparameter am Ein- oder Ausstiegspunkt sowie der geplanten Reihenfolge von Zustieg- und Ausstiegvorgängen, die sich aus der Tourenplanung ergibt. Entsprechende Parameter können jedoch auch im Rahmen der digitalen Buchung der Fahrt durch den Anwender und seinen hierbei definierten Präferenzen festgelegt werden. Eine Tour ist dabei als eine Zusammenstellung von mindestens zwei Fahraufträgen zu einem Gesamtfahrauftrag definiert. Durch die Reihenfolgeplanung der Auftragsabwicklung einer Tour leitet sich eine Routenplanung ab. Bei einer Tour kann somit eine zumindest zeitweise zeitgleiche Erfüllung von mehr als einem Fahrauftrag erfolgen und die fahrauftragsbezogenen Prozessschritte, die das Navigieren des Fortbewegungsmittels zu einem Einstiegspunkt, das Identifizieren des Insassen, das Einsteigen des Insassen, das fahrauftragsbezogene Transportieren zu einem Zielpunkt sowie das Aussteigen des Insassen sowie die hiermit einhergehenden Informations- und Kommunikationsprozesse umfassen, überlagern sich somit teilweise zu einer kumulierten, tourenbezogenen Gesamtprozesskette. Als eine sensorische Erfassung des zukünftigen Insassen ist insbesondere eine optische Vermessung des zukünftigen Insassen vor dem Einsteigen zu verstehen, aus der beispielsweise die Körpergröße ermittelt werden kann. Derart würde beispielsweise einem Kind vorzugsweise ein Sitzplatz zugeordnet, der sich nicht in der vorderen Sitzplatzreihe befindet. Zur sensorischen Erfassung wird vorzugsweise die Umgebungssensorik des Fortbewegungsmittels genutzt, worunter beispielsweise Kameras zur Straßenschildererkennung, Fußgängererkennung oder kamerabasierte Einpark-Unterstützungssysteme verstanden werden. Als vordefinierte Referenzen sind insbesondere spezifische Anforderungen des zukünftigen Insassen zu verstehen, mit anderen Worten vom Insassen formulierte Anforderungen an den Sitzplatz. Zur Formulierung von Anforderungen des Insassen an den Sitzplatz können entsprechende Eingabe- und/oder Auswahlfelder im Frontend eines digitalen Buchungssystems vorgesehen werden; es wäre auch eine Formulierung der Anforderungen mittels eines Vermittlers zum Beispiel an einem Buchungsschalter und/oder eine indirekte Formulierung der Anforderungen, beispielsweise mittels der Angabe von nicht im Gepäckfach zu transportierenden Ausrüstungsgegenständen und/oder der automatischen Erkennung der Mitführung von nicht im Gepäckfach mitzuführenden Ausrüstungsgegenständen denkbar. Nicht im Gepäckfach mitzuführende Ausrüstungsgegenstände können beispielsweise Handtaschen, kleinere Rucksäcke oder Schoßhunde sein. Die Sitzplatzzuordnung erfolgt unter Beachtung der zur Verfügung stehenden Sitzplätze und ihrer konkreten Sitzplatzparameter sowie unter Beachtung der übergeordneten Anforderungen aller Fahraufträge einer Tour mittels Auswahl eines Sitzplatzes, der die vordefinierten Referenzen am besten erfüllt. Die Sitzplatzzuordnung kann auf Grund aktueller Sitzplatzbelegungen oder unvorhersehbarer Ereignisse, worunter zum Beispiel die Einnahme eines anderen Sitzplatzes durch den Insassen und/oder eines vorher eingestiegenen Insassen und/oder die Verhinderung der Einnahme des Sitzplatzes verstanden werden kann, oder unvorhersehbarer Umfeldparameter am Ein- oder Ausstiegspunkt geändert werden.

Zur Erkennung des zukünftigen Insassen werden sensorbasiert durch das Fortbewegungsmittel biometrische Daten, worunter geometrische Daten, wie zum Beispiel der Augenabstand oder die Nasengröße oder ein Iris-Scan zu verstehen sind, und/oder nicht-geometrische Daten, wie zum Beispiel persönlich zuordenbare Bewegungsarten und/oder eindeutige persönliche Identifizierungsmerkmale herangezogen, die zur Identifikation mit Informationen aus einem bestehenden Datensatz verglichen werden oder mittels einer logischen und eineindeutigen Zuordnung eine Identifikation zulassen. Eindeutige persönliche Identifizierungsmerkmale können zum Beispiel in Form von Identifikationspapieren, insbesondere Personalausweis oder Führerschein, einer dem Insassen zugeordneten MAC Adresse, eines dem Insassen zugeordneten, tragbaren Anwenderendgerätes wie zum Beispiel ein Audio- oder Videogerät, ein Computer, insbesondere Laptop oder Tablet, ein Mobiltelefon, insbesondere Smartphone, ein Wearable Device, insbesondere Smartwatch, und/oder mittels der Auswertung einer Bluetooth-Kennung, Funkverbindung o.ä. des dem Insassen zugeordneten, tragbaren Anwenderendgerätes vorliegen. Es können zur Identifikation auch logische und/oder eineindeutige Zuordnungen mittels optischer Erkennungsmechanismen, wie beispielsweise die Darstellung einer spezifischen Farbe, eines Barcodes oder Musters auf einem dem Insassen zugeordneten, tragbaren Anwenderendgerätes, genutzt werden. Auf Basis der Identifikation eines Insassen erfolgt die logische Verknüpfung seiner persönlichen Daten mit dem ihm zugeordneten Sitzplatz. Biometrische Identifizierungsmerkmale können insbesondere sensorbasiert und/oder unter Verwendung von optischen Sensoren, beispielsweise kamerabasiert, ermittelt und mittels einer Auswerteeinheit weiterverarbeitet werden. Zur Verifikation persönlicher Identifizierungsmerkmale können unter anderem optische Sensoren zur Aufnahme von Bildern von zu verifizierenden Gegenständen oder die Auswertung von Verbindungen eines dem Insassen zugeordneten, tragbaren Anwenderendgerätes herangezogen werden. Die Nutzung optischer Erkennungsmechanismen basiert auf der Idee, dass vorzugsweise über eine digitale Systemplattform definierte Erkennungsmerkmale an ein dem Insassen zugeordnetes, tragbares Anwenderendgerät übermittelt werden, der diese spezifischen Merkmale zur Verifikation wiederum beispielsweise mittels optischer Sensoren digitalisiert und dem System zur Prüfung übergibt. Als ein nicht einschränkendes Beispiel kann ein Insasse in einer einem Fahrdienst/Fahrzeugpool/Taxidienst zugeordneten App ein spezifisches Merkmal übermittelt bekommen, zum Beispiel einen Barcode, den er mittels eines am Fahrzeug angebrachten Barcodescanners bzw. einer optischen Kamera zur Verifikation an das System übermittelt. Kann eine wartende Person nicht identifiziert werden, wird ein Signal ausgegeben, dass zur Information der wartenden Person und/oder zur Freischaltung einer alternativen Identifizierungsmethode und/oder zur Verhinderung eines Einstiegs eingerichtet ist, indem der wartenden Person der Zugang zu dem Fortbewegungsmittel verwehrt wird.

Die für die Ermittlung der aktuellen Position des Insassen genutzten Sensoren des Fortbewegungsmittels können für diese Anwendung zusätzlich integrierte oder bereits für andere Anwendungen genutzte Sensoren umfassen. Die Ermittlung der Positionsdaten ist insbesondere erforderlich, da die Nahfeldpositionierung des automatischen Fortbewegungsmittels sich insbesondere an der optimalen Erreichbarkeit des zugeordneten Sitzplatzes bzw. Gepäckfachs durch den zukünftigen und identifizierten Insassen ausrichtet. Die zur Positionsermittlung genutzten Systeme können zum Fortbewegungsmittel und/oder zu einem tragbaren Anwenderendgerät gehören und/oder einem externen System zugeordnet sein. Zur sensorbasierte Erfassung können Systeme genutzt werden, die einen Abstand zwischen dem Fortbewegungsmittel und dem zukünftigen Insassen ermitteln und die Positionsdaten in ein Koordinatensystem des Fortbewegungsmittels integrieren. Alternativ oder zusätzlich können globale Positionsdaten ermittelt werden, wie beispielsweise sattelitengestützt ermittelte Positionsdaten oder funktechnisch ermittelte Positionsdaten, wobei beispielsweise eine Ermittlung der Daten mittels Kombination von LoRaWAN (Low Power Wide Area Network) und BLE (Bluetooth Low Energy) oder unter Anwendung einer Lokalisierung mittels RSSI (Received Signal Strength Indication) erfolgen kann. Zur sensorbasierten Positionsbestimmung können insbesondere optische Sensoren, vorzugsweise eine optische Kamera, wobei vorzugsweise eine Front- und/oder eine Seitenkamera, die beispielsweise in den Außenspiegel integriert ist bzw. sind, genutzt werden, oder Ultraschallsensoren herangezogen werden. Auch die Verwendung eines Radars, eines Lidar-Sensors, eines Laser-Distanzmessgerätes oder eine Datenauswertung eines vom zukünftigen Insassen mitgeführten, tragbaren Anwenderendgerätes oder einer App auf einem vom Insassen mitgeführten, tragbaren Anwenderendgerät ist möglich. Eine Informationsfusion verschiedener Datenquellen und/oder eine Sensorfusion können insbesondere zur Optimierung der Genauigkeit der Positionsdaten vorgesehen sein.

Zur Kennzeichnung des zugeordneten und damit vorbestimmten Sitzplatzes wird vorzugsweise ein Signal ausgegeben, dass drahtlos oder kabelgebunden sein kann und zumindest einen Aktor anspricht, der den zukünftigen Insassen über den für ihn vorgesehenen Sitzplatz informiert. Die Anzeige des zugeordneten Sitzplatzes kann mittels des Fortbewegungsmittels und/oder mittels externer Systeme und/oder mittels Zusammenspiel zwischen externen Systemen und dem Fortbewegungsmittel erfolgen. Als externes System zur Anzeige des zugeordneten Sitzplatzes kann vorzugsweise ein dem Insassen zugeordnetes, tragbares Anwenderendgerät genutzt werden, auf dem beispielsweise mittels einer textuellen Information und/oder einer schematischen Darstellung eine eindeutige Kennzeichnung der anzustrebenden Einstiegsposition erfolgt. Bei einem Zusammenspiel eines externen Systems, beispielsweise in Form eines dem Insassen zugeordneten, tragbaren Anwenderendgerätes, und des Fortbewegungsmittels wäre beispielsweise die Anwendung einer farblichen Kodierung vorstellbar, bei der die Einstiegspositionen des Fortbewegungsmittels mit entsprechenden Farbmarkern ausgestattet sind, und die entsprechende Farbe zum bevorstehenden Einstiegszeitpunkt auch auf dem dem Insassen zugeordneten, tragbaren Anwenderendgerät angezeigt wird. Eine Kennzeichnung am Fortbewegungsmittel kann vorzugsweise durch eine textuelle Information oder schematische Darstellung auf einem zentralen Display erfolgen. Das Display kann zusätzlich oder alternativ einem Einstiegspunkt konkret zuordenbar sein. Weiterhin sollte das Display vorzugsweisegut sichtbar für den zukünftigen Insassen angeordnet sein. Alternativ oder zusätzlich kann ein akustisches Signal erfolgen, welches vorzugsweise über einen Lautsprecher ausgegeben wird. Schließlich kann auch eine eindeutige Markierung der Einstiegsposition erfolgen, worunter beispielsweise eine optische, farbliche und/oder formbezogene Markierung, die vorzugsweise als Türbeleuchtung oder LED-Untergrundbeleuchtung ausgestaltet sein kann, verstanden wird. Es kann alternativ oder zusätzlich eine Kommunikationsschnittstelle zwischen dem Fortbewegungsmittel und den Insassen vorgesehen sein. Diese kann zentral oder dezentral angeordnet sein und vorzugsweise mittels eines einem Insassen zugeordneten, tragbaren Anwenderendgerätes und/oder eines Displays eine Kommunikation zwischen Systemen des Fortbewegungsmittels und dem Insassen realisieren. Weiterhin kann eine Sprachsteuerung und/oder gestenbasierte Steuerung vorgesehen sein. Die Kennzeichnung des zugeordneten Sitzplatzes kann zusätzlich oder alternativ auch innerhalb des Fortbewegungsmittels erfolgen, wobei hier ähnliche oder gleiche Systeme wie im Außenbereich eingesetzt werden können. Das Signal zur Anzeige des zugeordneten Sitzplatzes kann von einem externen System weiterverarbeitet werden, indem beispielsweise ein Wegfindungssystem eines elektrischen Rollstuhls die Position des vorbestimmten Einstiegspunktes als Zielparameter verarbeitet. Das Signal kann zusätzlich oder alternativ zur Information der im Fortbewegungsmittel bereits anwesenden Insassen vorgesehen sein und/oder den Einstieg des Insassen in das Fortbewegungsmittel technisch ermöglichen, indem beispielsweise eine Türverriegelung angesprochen wird. Zum Ende einer insassenspezifischen Fahrt wird vorzugsweise ein Signal ausgegeben. Dieses kann kabelgebunden oder drahtlos vermittelt werden und ist ebenfalls vorzugsweise zur Information des Insassen vorgesehen, wobei dieser hiermit über das Erreichen seines Zielpunktes informiert ist. Die Information kann vorzugsweise mittels eines optischen Signals in Form einer farblichen Kodierung oder textuelle Information mittels eines Displays im Fortbewegungsmittel erfolgen. Das Display kann zentral oder dezentral angeordnet sein. Zusätzlich oder alternativ kann ein akustisches Signal den Insassen über das Erreichen seines Zielpunktes informieren. Dieses kann beispielsweise über die Lautsprecher des Audiosystems des Fortbewegungsmittels oder ein sitzplatzspezifisches Audiosystem ausgegeben werden. Schließlich kann der Insasse über die Zielerreichung auch mittels einer Anzeige über ein ihm zugeordnetes, tragbares Anwenderendgerät informiert werden. Zudem kann das Signal eingerichtet sein, bei Erreichen eines insassenspezifischen Zielpunktes den nächstliegenden und/oder vorzugsweise zu nutzenden Ausstiegspunkt mittels gleicher oder ähnlicher Systeme wie zur Anzeige des Erreichens eines insassenspezifischen Zielpunktes beschrieben anzuzeigen. Weiter kann das Signal eingerichtet sein, zum Verlassen des Fortbewegungsmittels aufzufordern und/oder das Verlassen des Fortbewegungsmittels technisch zu ermöglichen, also beispielsweise eine Türverriegelung aufzuheben und/oder eine Tür automatisch zu öffnen und/oder das Gepäckfach zu entriegeln und/oder selbsttätig zu öffnen, wenn dem Insassen hierin transportierte Ausrüstungsgegenstände zugeordnet sind.

Gemäß einem zweiten Aspekt wird eine Vorrichtung zum Positionieren eines automatisiert fahrfähigen Fortbewegungsmittels vorgeschlagen. Die Vorrichtung kann dauerhaft in dem Fortbewegungsmittel integriert sein. Sie umfasst einen Dateneingang, welcher beispielsweise einen Busteilnehmer umfassen kann. Über den Dateneingang kann beispielsweise eine Datenverbindung zu einem internen oder externen Datenspeicher aufgebaut werden. Zusätzlich oder alternativ kann hierüber eine Datenverbindung zu einem elektronischen Steuergerät hergestellt werden. Schließlich können über den Dateneingang auch ein digitales Sensorsignal und/oder ein drahtloses Signal eines Anwenderendgerätes empfangen werden. Die informationstechnische Anbindung an den Dateneingang kann drahtlos oder drahtgebunden erfolgen. Mittels des Dateneingangs kann zusätzlich oder alternativ eine Bluetooth- und/oder W-LAN-Verbindung zu einem tragbaren Anwenderendgerät aufgebaut werden. Die Verbindung zwischen dem tragbaren Anwenderendgerät und dem Fortbewegungsmittel kann auch zum Zwecke der vorliegenden Erfindung internetvermittelt sein. Weiter ist eine Auswerteeinheit in der Vorrichtung vorgesehen, welche als programmierbarer Prozessor, Mikrokontroller und/oder elektronisches Steuergerät ausgestaltet sein kann. Durch die Auswerteeinheit wird die Vorrichtung in die Lage versetzt, die logischen Schritte des in Verbindung mit dem erstgenannten Erfindungsaspekt ausgeführten Verfahrens auszuführen. Weiter ist ein Datenausgang vorgesehen, welcher informationstechnisch mit der Auswerteeinheit gekoppelt ist. Die Auswerteeinheit ist eingerichtet, mittels des Dateneingangs einen zukünftigen Insassen automatisch zu erkennen sowie mittels Auswertung der Sensordaten einer Umgebungssensorik seine aktuelle Position zu ermitteln und eine optimale Positionierung des Fortbewegungsmittels in Bezug zu der aktuellen Position des zukünftigen Insassen festzulegen, die einen besseren Zugriff auf eine vordefinierte Tür des Fortbewegungsmittels gegenüber anderen Türen ermöglicht. Ein besserer Zugriff kann sich durch eine bessere Erreichbarkeit auszeichnen, worunter eine kurze Strecke zwischen der aktuellen Position des zukünftigen Insassen und der vordefinierten Tür verstanden wird. In die Festlegung der Positionierung können vordefinierte Referenzen eingehen, worunter beispielsweise fahrzeugspezifische Parameter, insbesondere Geometrien und Abmessungen des Fortbewegungsmittels, oder Umgebungsparameter des Ein- oder Ausstiegspunktes verstanden werden. In Verbindung mit dem Datenausgang ist die Auswerteeinheit weiter eingerichtet, automatisch ein Signal zur Umsetzung der Positionierung des Fortbewegungsmittels auszugeben.

Die Merkmale, Merkmalskombination und die sich aus diesen ergebenden Vorteile der erfindungsgemäßen Vorrichtung und ihrer bevorzugten Ausgestaltungen entsprechen derart ersichtlich denjenigen, welche in Verbindung mit dem oben genannten Verfahren ausgeführt worden sind, dass zur Vermeidung von Wiederholungen auf die obigen Ausführungen verwiesen wird.

Gemäß einem dritten Aspekt wird ein Fortbewegungsmittel (z. B. ein PKW, Transporter, LKW, Motorrad, Land- und/oder Wasserfahrzeug) vorgeschlagen, welches eine Vorrichtung gemäß dem zweitgenannten Erfindungsaspekt und des Unteranspruchs umfasst. Auch bezüglich des Fortbewegungsmittels wird hinsichtlich der Merkmale, Merkmalskombinationen und Vorteile auf die obigen Ausführungen verwiesen, um Wiederholungen zu vermeiden.

Nachfolgend wird auf beispielhafte Ausgestaltungen und einzelne Merkmale der vorliegenden Erfindung ohne einschränkenden Charakter eingegangen. Beispielsweise erwartet ein zukünftiger Insasse das Eintreffen eines automatisch fahrenden PKWs. Bei der tourenspezifischen Planung wurde dem Insassen unter Beachtung seiner Buchungswünsche und in Einklang mit den tourenspezifischen Fahraufträgen ein spezifischer Sitzplatz zugeordnet, der sich hinter dem Beifahrersitz befindet. Während des Eintreffens des PKW am Einstiegspunkt wird seine Identität durch die Auswertung der funktechnischen Verbindung des tragbaren Anwenderendgerätes des wartenden, zukünftigen Insassen verifiziert und die Position des wartenden, zukünftigen Insassen sensorisch durch das Fortbewegungsmittel erfasst. Hierzu wird eine im Außenspiegel des Fortbewegungsmittels installierte Kamera genutzt. Es wird festgestellt, dass der zukünftige Insasse keine Ausrüstungsgegenstände mit sich führt. Im Ansprechen auf die Identifikation und die logische Verknüpfung mit dem zuzuordnenden Sitzplatz sowie unter Berücksichtigung der festgestellten Position des wartenden, zukünftigen Insassen und der fahrzeugspezifischen Geometrie und Abmessungen erfolgt eine Festlegung und Umsetzung der optimalen Positionierung des Fortbewegungsmittels neben dem wartenden, zukünftigen Insassen. Auf Grund der Zuordnung des Sitzplatzes kommt das Fortbewegungsmittel in diesem Ausführungsbeispiel also mit der rechten C-Säule neben dem wartenden, zukünftigen Insassen zum Stehen. Die zu nutzende Tür wird dem wartenden, zukünftigen Insassen in Textform auf dem der Tür zugehörigen Display angezeigt. Der wartende Insasse öffnet die Tür und begibt sich auf seinen Sitzplatz. Nach dem Transport und bei Erreichen eines vordefinierten Ausstiegspunktes wird der Insasse per Lautsprecher über die Zielerreichung und den für ihn anstehenden Ausstiegvorgang informiert und die vorbestimmte Tür hinten rechts geöffnet. Durch die insassensitzplatzspezifische und bedürfnisorientierte Positionierung des PKWs beim Ein- und Ausstiegvorgang kann die Behaglichkeit und damit Akzeptanz der Nutzung automatisch fahrenden Fortbewegungsmittel erhöht werden.

Weitere Einzelheiten, Merkmale und Vorteile der Erfindung ergeben sich aus der nachfolgenden Beschreibung und den Figuren. Es zeigen:
- Figur 1: eine schematische Darstellung eines Ausführungsbeispiels eines erfindungsgemäßen Fortbewegungsmittels, umfassend ein Ausführungsbeispiel einer erfindungsgemäßen Vorrichtung; und
- Figur 2: ein Flussdiagramm veranschaulichend Schritte eines Ausführungsbeispiels eines erfindungsgemäßen Verfahrens zur insassensitzplatzspezifischen Positionierung eines automatisch fahrenden Fortbewegungsmittels.

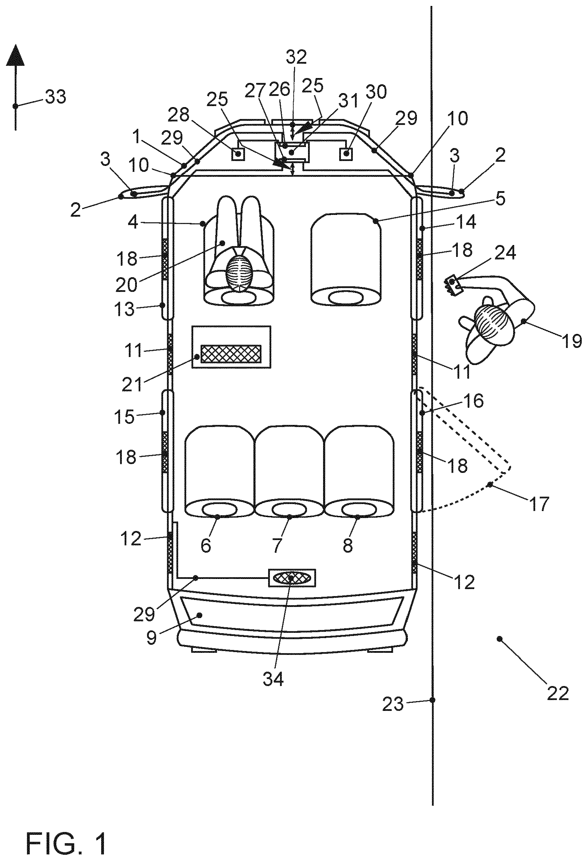

Figur 1 zeigt einen PKW 1 als Ausführungsbeispiel eines erfindungsgemäßen Fortbewegungsmittels, in welchem eine Vorrichtung in Form eines elektronischen Steuergerätes 31 als Auswerteeinheit umfassend einen Dateneingang 26 und einen Datenausgang 27 installiert sind. Der Dateneingang 26 ist mit einer Antenne 25 mit dem Smartphone 24 als Ausführungsbeispiel eines tragbaren Anwenderendgerätes eines auf einem Bürgersteig 22 wartenden, zukünftigen Insassen 19 informationstechnisch verbunden und tauscht ein Drahtloskommunikationssignal 32 mit der Antenne 25 aus. Weiter ist der Dateneingang 26 mit in den Außenspiegeln 2 integrierten Kameras 3 und mit einem elektronischen Steuergerät 29 sowie einem Datenspeicher 30 informationstechnisch verbunden und tauscht mit diesen ein kabelgebunden übertragenes Signal 29 aus. Der Datenausgang 27 ist informationstechnisch mit an den Türen 13, 14, 15, 16 befindlichen Displays 18, mittels einer Antenne 25 mit dem Smartphone 24 des wartenden, zukünftigen Insassen 19 sowie mittels eines kabelgebunden übertragenen Signals 29 mit einem Lautsprecher 34 sowie einem Datenspeicher 30 und einem elektronischen Steuergerät 28 verbunden. Der PKW verfügt über vier Türen 13, 14, 15, 16 sowie fünf Sitzplätze 4, 5, 6, 7, 8 und ein mittels Kofferraumklappe 9 zugängliches Gepäckfach. Vor den vorderen Türen 13, 14 ist beidseitig eine A-Säule 10 angeordnet, zwischen den vorderen 13, 14 und hinteren Türen 15, 16 ist beidseitig eine B-Säule 11 angeordnet, hinter den hinteren Türen 15, 16 ist beidseitig eine C-Säule 12 angeordnet, anhand derer Position eine geometrische Beschreibung des PKWs 1 möglich ist. In diese geht zur Ableitung spezifischer Einstiegspositionen das Öffnungsfeld 17 der jeweiligen Tür ein, welches hier für Tür D 16 exemplarisch dargestellt ist. Auf dem Sitzplatz A 4, der sich vorne links im PKW befindet, sitzt ein Insasse 20. Sitzplatz B 5, Sitzplatz C 6, Sitzplatz D 7 sowie Sitzplatz E 8 sind derzeit unbesetzt. Ein Tisch 21 stellt eine spezifische Ausrüstung des Sitzplatzes C 6 dar. Eine vorwärts gerichtete Fahrtrichtung 33 richtet sich an der Längsachse des PKWs 1 aus und beschreibt einen Bewegungsvektor in Blickrichtung der Insassen 20. In einem Datenspeicher 30 werden Informationen zu den erwarteten Insassen und dem zuzuordnenden Sitzplatz 4, 5, 6, 7, 8 vorgehalten und über den Dateneingang an die Auswerteeinheit 31 übermittelt. Das Erkennen eines zukünftigen Insassen 19 erfolgt mittels Auswertung eines vom Smartphone 24 des auf einem Bürgersteig 22 wartenden, zukünftigen Insassen 19 ausgesendeten Drahtloskommunikationssignal 32 das mittels Antenne 25 am Dateneingang 26 der Auswerteeinheit 31 eingeht. Die genaue Position des wartenden, zukünftigen Insassen 19 wird von der Auswerteeinheit 31 durch Auswertung von als kabelgebundenes Signal 29 übermittelten Bildinformationen ermittelt, die mittels einer am Außenspiegel 2 befindlichen Kamera 3 als Ausführungsbeispiel eines optischen Sensors aufgenommen werden. Basierend auf der Erkennung des zukünftigen Insassen erfolgt die logische Zuordnung des zugeordneten Sitzplatzes 4, 5, 6, 7, 8, in diesem Beispiel Sitzplatz E 8. Im Ansprechen hierauf und auf Grund der Tatsache, dass sensorisch durch die am Außenspiegel 2 befindlichen Kamera 3 als Ausführungsbeispiel eines optischen Sensors keine Ausrüstungsgegenstände erkannt wurden, wird über den Datenausgang 27 ein Signal 29 ausgegeben, auf Basis dessen die für den Einstieg vorgesehene Tür D 16 mittels einer visuellen Einblendung auf einem an der Tür D 16 befindlichen Displays 18 gekennzeichnet wird. Des Weiteren erfolgt durch die Auswerteeinheit 31 eine Festlegung der Positionierung mittels Berechnung der optimalen Halteposition des PKWs 1 zur Erzielung eines möglichst kurzen Weges zwischen der Position des wartenden, zukünftigen Insassen 19 und dem vorbestimmten Einstiegspunkt unter Beachtung des dem wartenden, zukünftigen Insassen 19 zugeordneten Sitzplatzes E 8 und der diesem Sitzplatz zugeordneten nächstliegenden Tür D 16 sowie des fahrzeuggeometriespezifischen Türöffnungsfeldes 17 und von Umfeldparametern. Die Positionierungsdaten werden zur weiteren Verarbeitung über den Datenausgang 27 an ein elektronisches Steuergerät 28 übermittelt. Somit wird der PKW 1 erfindungsgemäß optimal zur Erreichung eines zugeordneten Sitzplatzes E 8 durch den wartenden, zukünftigen Insassen 19 positioniert, um derart seinen Komfort beim Einstiegsvorgang der Nutzung eines automatischen PKWs 1 zu erhöhen.

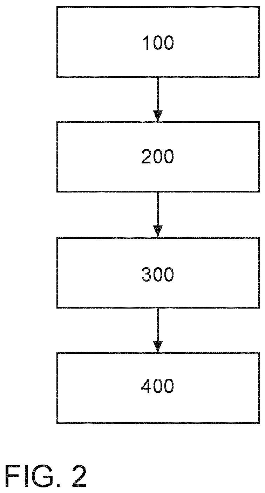

Figur 2 zeigt ein Flussdiagramm veranschaulichend Schritte eines Ausführungsbeispiels eines erfindungsgemäßen Verfahrens zum insassensitzplatzspezifischen Positionieren eines automatischen Fortbewegungsmittels. In einem ersten Schritt 100 wird automatisch ein zukünftiger Insasse erkannt. Hierfür kommen insbesondere Sensorinformationen und digitale Verbindungsinformationen tragbarer Anwenderendgeräte eines Insassen zum Einsatz. In Schritt 200 wird die aktuelle Position des Insassen automatisch ermittelt. Zur Ermittlung der aktuellen Position werden insbesondere Sensoren und Positionsdaten tragbarer Anwenderendgeräte herangezogen. Auf Basis dieser Informationen wird in Schritt 300 die optimale Positionierung in Bezug zu der aktuellen Position des zukünftigen Insassen und anhand einer vordefinierten Referenz automatisch festgelegt und im Ansprechen hierauf in Schritt 400 ein Signal ausgegeben, was zur Umsetzung der Positionierung sowie der Anzeige der vorbestimmten Einstiegsposition eingerichtet ist. Im Ergebnis wird ein Fortbewegungsmittel durch das erfindungsgemäße Verfahren optimal in Bezug auf die Anforderungen eines wartenden, zukünftigen Insassen positioniert, um derart den Komfort des Einstiegsvorgangs bei der Nutzung eines automatischen Fortbewegungsmittels zu erhöhen.

### Bezugszeichenliste

- 1: PKW
- 2: Außenspiegel
- 3: Kamera
- 4: Sitzplatz A
- 5: Sitzplatz B
- 6: Sitzplatz C
- 7: Sitzplatz D
- 8: Sitzplatz E
- 9: Kofferraumklappe
- 10: A-Säule
- 11: B-Säule
- 12: C-Säule
- 13: Tür A
- 14: Tür B
- 15: Tür C
- 16: Tür D
- 17: Öffnungsfeld der Tür 4
- 18: Display
- 19: Wartender, zukünftiger Insasse
- 20: Insasse
- 21: Tisch
- 22: Bürgersteig
- 23: Fahrbahnrand
- 24: Smartphone
- 25: Antenne
- 26: Dateneingang
- 27: Datenausgang
- 28: Elektronisches Steuergerät
- 29: Signal
- 30: Datenspeicher
- 31: Auswerteeinheit
- 32: Drahtloskommunikationssignal
- 33: Fahrtrichtung
- 34: Lautsprecher

## Patentansprüche

1. Verfahren zum Positionieren eines automatisiert fahrfähigen Fortbewegungsmittels (1), umfassend die Schritte:
- automatisches Erkennen (100) eines zukünftigen Insassen (19),
- automatisches Ermitteln (200) einer aktuellen Position des zukünftigen Insassen (19) mittels einer Umgebungssensorik,
- automatisches Festlegen (300) einer optimalen Positionierung des Fortbewegungsmittels (1) für einen Zugriff auf eine vordefinierte Tür des Fortbewegungsmittels (1) gegenüber anderen Türen des Fortbewegungsmittels (1) in Bezug zu der aktuellen Position des zukünftigen Insassen (19) und im Ansprechen hierauf
- Ausgeben (400) eines Signals (29) zur Positionierung des Fortbewegungsmittels (1), **dadurch gekennzeichnet, dass** eine Zuordnung eines Sitzplatzes auf Basis einer sensorischen Vermessung des zukünftigen Insassen (19) mittels des Fortbewegungsmittels (1) erfolgt.

2. Verfahren nach Anspruch 1, weiter umfassend
- automatisches Ermitteln eines dem zukünftigen Insassen (19) zugeordneten Sitzplatzes (8) und
- automatisches Berücksichtigen
- des zukünftigen Sitzplatzes (8) und/der
- der Erreichbarkeit des zugeordneten Sitzplatzes (8) und/oder
- der Fahrzeuggeometrie und/oder
- von Umgebungsparametern des Ein- und/oder Ausstiegspunktes
bei der Positionierung.

3. Verfahren nach Anspruch 2, wobei der Sitzplatz (8) einer Sitzreihe zugeordnet ist und die Positionierung in Abhängigkeit von der Sitzreihe erfolgt.

4. Verfahren nach einem der vorhergehenden Ansprüche, weiter umfassend
- automatisches Ermitteln eines dem zukünftigen Insassen (19) zugehörigen Ausrüstungsgegenstandes und Berücksichtigen des zugehörigen Ausrüstungsgegenstandes bei der Positionierung.

5. Verfahren nach Anspruch 4, wobei der Ausrüstungsgegenstand ein Gepäckstück ist und die Positionierung in Abhängigkeit von der Position eines Gepäckfaches des Fortbewegungsmittels erfolgt.

6. Verfahren nach einem der vorstehenden Ansprüche, wobei im Ansprechen auf die Positionierung eine Tür entriegelt, insbesondere selbsttätig geöffnet, wird, die der Positionierung logisch zugeordnet ist.

7. Verfahren nach einem der vorherigen Ansprüche, wobei eine Zuordnung eines Sitzplatzes (8)
- automatisch erfolgt und/oder
- auf Basis vordefinierter Referenzen erfolgt und/oder
- auf Basis von Umfeldparametern am Einstiegs- und/oder Ausstiegspunkt erfolgt und/oder
- auf Basis einer geplanten Reihenfolge von Zu- und Ausstiegsvorgängen erfolgt.

8. Verfahren nach einem der vorherigen Ansprüche, wobei die Erkennung des zukünftigen Insassen (19) auf Basis biometrischer Daten und/oder eindeutiger persönlicher Identifizierungsmerkmale erfolgt, die sensorbasiert durch das Fortbewegungsmittel (1) ermittelt und mit gespeicherten Datensätzen abgeglichen werden.

9. Verfahren nach einem der vorstehenden Ansprüche, wobei die Ermittlung der aktuellen Position des zukünftigen Insassen (19)
- sensorbasiert mittels eines optischen Sensors (3), vorzugsweise mittels einer optischen Kamera, und/oder
- mittels eines Ultraschallsensors und/oder
- mittels eines Radarsensors und/oder
- mittels Verwendung eines Lidar-Sensors und/oder
- mittels eines Laser-Distanzmessgerätes und/oder,
- mittels Datenauswertung eines vom Insassen (19) mitgeführten, tragbaren Anwenderendgerätes (24) und/oder
- mittels einer App auf einem vom Insassen (19) mitgeführten, tragbaren Anwenderendgerätes (24)
erfolgt.

10. Verfahren nach einem der vorstehenden Ansprüche, wobei ein drahtloses oder kabelgebundenes Signal (29) zur Kennzeichnung eines Sitzplatzes (8) ausgegeben wird, welches die Kennzeichnung des Sitzplatzes (8)
- als farbliche Kodierung und/oder
- als textuelle Information und/oder
- als optisches und/oder
- als akustisches Signal und/oder
- als schematische Darstellung und/oder
- auf einem tragbaren Anwenderendgerät (24) und/oder
- auf einem Display (18) des Fortbewegungsmittels (1) und/oder
- als eine eineindeutige Markierung der Einstiegsposition
veranlasst.

11. Vorrichtung zum Positionieren eines automatisiert fahrfähigen Fortbewegungsmittels (1), umfassend:
- einen Dateneingang (26),
- eine Auswerteeinheit (31),
- einen Datenausgang (27); und
- eine Umgebungssensorik;
wobei die Auswerteeinheit (31) eingerichtet ist,
- mittels des Dateneingangs (26)
- einen zukünftigen Insassen (19) automatisch zu erkennen,
- die aktuelle Position des zukünftigen Insassen (19) automatisch mittels einer Umgebungssensorik zu ermitteln und
- eine optimale Positionierung des Fortbewegungsmittels (1) für einen Zugriff auf eine vordefinierte Tür des Fortbewegungsmittels gegenüber anderen Türen des Fortbewegungsmittels in Bezug zu der aktuellen Position des zukünftigen Insassen (19) festzulegen, und im Ansprechen darauf
- in Verbindung mit dem Datenausgang (27) ein Signal (29) zur Umsetzung der Positionierung des Fortbewegungsmittels (1) auszugeben,
**dadurch gekennzeichnet, dass**
- eine Zuordnung eines Sitzplatzes auf Basis einer sensorischen Erfassung eines zukünftigen Insassen (19) mittels des Fortbewegungsmittels (1) erfolgt.

12. Vorrichtung nach Anspruch 11, welche eingerichtet ist, ein Verfahren nach einem der Ansprüche 2 bis 10 auszuführen.

13. Fortbewegungsmittel (1) umfassend eine Vorrichtung nach Anspruch 11 oder 12.

## Claims

1. Method for positioning an automatically operable means of transport (1), comprising the steps of:
- automatically detecting (100) a future occupant (19),
- automatically identifying (200) a current position of the future occupant (19) by means of an environmental sensor system,
- automatically determining (300) optimal positioning of the means of transport (1) for access to a predefined door of the means of transport (1) relative to other doors of the means of transport (1) in relation to the current position of the future occupant (19), and, in response to this,
- outputting (400) a signal (29) for positioning the means of transport (1), **characterized in that** a seat is allocated on the basis of a sensor measurement of the future occupant (19) by means of the means of transport (1).

2. Method according to claim 1, further comprising
- automatically identifying a seat (8) allocated to the future occupant (19), and
- automatically taking into consideration
- the future seat (8), and/or
- the accessibility of the allocated seat (8), and/or
- the vehicle geometry, and/or
- environmental parameters of the entry and/or exit point during positioning.

3. Method according to claim 2, wherein the seat (8) is allocated to a row of seats and the positioning is carried out depending on the row of seats.

4. Method according to any of the preceding claims, further comprising
- automatically identifying an item of equipment associated with the future occupant (19) and taking into account the associated item of equipment during positioning.

5. Method according to claim 4, wherein the item of equipment is a piece of luggage and the positioning is carried out depending on the position of a luggage compartment of the means of transport.

6. Method according to any of the preceding claims, wherein, in response to the positioning, a door which is logically allocated to the positioning is unlocked, in particular automatically opened.

7. Method according to any of the preceding claims, wherein a seat (8) is allocated
- automatically, and/or
- on the basis of predefined references, and/or
- on the basis of environmental parameters at the entry and/or exit point, and/or
- on the basis of a planned sequence of boarding and disembarkation processes.

8. Method according to any of the preceding claims, wherein the future occupant (19) is detected on the basis of biometric data and/or unique personal identification features which are identified by sensor by the means of transport (1) and compared with stored data records.

9. Method according to any of the preceding claims, wherein the current position of the future occupant (19) is identified
- by sensor by means of an optical sensor (3), preferably by means of an optical camera, and/or
- by means of an ultrasonic sensor, and/or
- by means of a radar sensor, and/or
- by using a LIDAR sensor, and/or
- by means of a laser distance measuring apparatus, and/or
- by means of data evaluation of a portable user terminal (24) carried by the occupant (19), and/or
- by means of an app on a portable user terminal (24) carried by the occupant (19).

10. Method according to any of the preceding claims, wherein a wireless or wired signal (29) is output to identify a seat (8), which signal causes the seat (8) to be identified
- with color coding, and/or
- with textual information, and/or
- with an optical and/or
- with an acoustic signal, and/or
- with a schematic depiction, and/or
- on a portable user terminal (24), and/or
- on a display (18) of the means of transport (1), and/or
- with a unique marking of the entry position.

11. Device for positioning an automatically operable means of transport (1), comprising:
- a data input (26),
- an evaluation unit (31),
- a data output (27), and
- an environmental sensor system,
the evaluation unit (31) being designed,
- by means of the data input (26),
- to automatically detect a future occupant (19),
- to automatically identify the current position of the future occupant (19) by means of an environmental sensor system, and
- to determine optimal positioning of the means of transport (1) for access to a predefined door of the means of transport relative to other doors of the means of transport in relation to the current position of the future occupant (19), and, in response to this,
- in conjunction with the data output (27), to output a signal (29) for carrying out the positioning of the means of transport (1),
**characterized in that**
- a seat is allocated on the basis of a sensor recording of a future occupant (19) by means of the means of transport (1).

12. Device according to claim 11, which is designed to perform a method according to any of claims 2 to 10.

13. Means of transport (1) comprising a device according to claim 11 or 12.

## Revendications

1. Procédé permettant de positionner un moyen de locomotion (1) capable d'une conduite automatisée, comprenant les étapes consistant à :
- reconnaître automatiquement (100) un futur occupant (19),
- déterminer automatiquement (200) une position actuelle du futur occupant (19) à l'aide d'un système de capteurs environnementaux,
- établir automatiquement (300) un positionnement optimal du moyen de locomotion (1) pour un accès à une porte prédéfinie du moyen de locomotion (1) par rapport à d'autres portes du moyen de locomotion (1) par rapport à la position actuelle du futur occupant (19) et en réponse à celle-ci
- émettre (400) un signal (29) pour le positionnement du moyen de locomotion (1), **caractérisé en ce qu'**une attribution d'une place assise est effectuée sur la base d'un mesurage par capteur du futur occupant (19) à l'aide du moyen de locomotion (1).

2. Procédé selon la revendication 1, comprenant en outre
- la détermination automatique d'une place assise (8) attribuée au futur occupant (19), et
- la prise en compte automatique
- de la future place assise (8) et/ou
- de l'accessibilité de la place assise (8) attribuée et/ou
- de la géométrie de véhicule et/ou
- de paramètres environnementaux du point d'entrée et/ou de sortie lors du positionnement.

3. Procédé selon la revendication 2, dans lequel la place assise (8) est attribuée à une rangée de sièges et le positionnement est effectué en fonction de la rangée de sièges.

4. Procédé selon l'une des revendications précédentes, comprenant en outre
- la détermination automatique d'une pièce d'équipement associée au futur occupant (19) et la prise en compte de la pièce d'équipement associée lors du positionnement.

5. Procédé selon la revendication 4, dans lequel la pièce d'équipement est un bagage et le positionnement est effectué en fonction de la position d'un compartiment à bagages du moyen de locomotion.

6. Procédé selon l'une des revendications précédentes, dans lequel, en réponse au positionnement, une porte, qui est logiquement associée au positionnement, est déverrouillée, en particulier ouverte automatiquement.

7. Procédé selon l'une des revendications précédentes, dans lequel une attribution d'une place assise (8)
- s'effectue automatiquement et/ou
- s'effectue sur la base de références prédéfinies et/ou
- s'effectue sur la base de paramètres environnementaux au point d'entrée et/ou de sortie et/ou
- s'effectue sur la base d'une séquence planifiée d'opérations d'entrée et de sortie.

8. Procédé selon l'une des revendications précédentes, dans lequel la reconnaissance du futur occupant (19) s'effectue sur la base de données biométriques et/ou de caractéristiques d'identification personnelles univoques, qui sont déterminées par le moyen de locomotion (1) sur la base de capteurs et sont comparées à des jeux de données mémorisés.

9. Procédé selon l'une des revendications précédentes, dans lequel la détermination de la position actuelle du futur occupant (19) s'effectue
- sur la base de capteurs à l'aide d'un capteur optique (3), de préférence à l'aide d'une caméra optique, et/ou
- à l'aide d'un capteur à ultrasons et/ou
- à l'aide d'un capteur radar et/ou
- à l'aide d'un capteur lidar, et/ou
- à l'aide d'un télémètre laser et/ou,
- à l'aide d'une évaluation de données d'un terminal d'utilisateur portatif (24) emporté par l'occupant (19) et/ou
- à l'aide d'une application sur un terminal d'application portatif (19) emporté par l'occupant (24).

10. Procédé selon l'une des revendications précédentes, dans lequel un signal sans fil ou câblé (29) est émis pour l'identification d'une place assise (8), lequel provoque l'identification de la place assise (8)
- sous la forme d'un code couleur et/ou
- sous la forme d'informations textuelles et/ou
- sous la forme d'un signal optique et/ou
- sous la forme d'un signal sonore et/ou
- sous la forme d'une représentation schématique et/ou
- sur un terminal d'utilisateur portatif (24) et/ou
- sur un afficheur (18) du moyen de locomotion (1) et/ou
- sous la forme d'un marquage univoque de la position d'entrée.

11. Dispositif permettant de positionner un moyen de locomotion (1) capable d'une conduite automatisée, comprenant :
- une entrée de données (26),
- une unité d'évaluation (31),
- une sortie de données (27) ; et
- un système de capteurs environnementaux ;
dans lequel l'unité d'évaluation (31) est conçue pour,
- au moyen de l'entrée de données (26)
- reconnaître automatiquement un futur occupant (19),
- déterminer automatiquement la position actuelle du futur occupant (19) à l'aide d'un système de capteurs environnementaux, et
- établir un positionnement optimal du moyen de locomotion (1) pour un accès à une porte prédéfinie du moyen de locomotion par rapport à d'autres portes du moyen de locomotion par rapport à la position actuelle du futur occupant (19), et en réponse à cela
- émettre, en liaison avec la sortie de données (27), un signal (29) pour la conversion du positionnement du moyen de locomotion (1),
**caractérisé en ce**
- **qu'**une attribution d'une place assise est effectuée sur la base d'une détection par capteur d'un futur occupant (19) à l'aide du moyen de locomotion (1).

12. Dispositif selon la revendication 11, lequel est conçu pour exécuter un procédé selon l'une quelconque des revendications 2 à 10.

13. Moyen de locomotion (1) comprenant un dispositif selon la revendication 11 ou 12.
